# EUROPEAN PATENT APPLICATION

(11) **EP 3 153 157 A1**
(43) Date of publication of application: **12.04.2017**
(21) Application number: 15189168.6
(22) Date of filing: 09.10.2015
(51) Int. Cl.: A61K 9/16, A61K 9/20, A61K 31/551

(54) **PHARMACEUTICAL COMPOSITION FOR PROLONGED RELEASE OF NEVIRAPINE**

(71) Applicant: Teva Pharmaceutical Works Private Limited Company, 4042 Debrecen (HU)
(72) Inventor: SKALICZKI, Timea Dr., 4080 Hajdúnánás (HU); TÖRZSÖK, Brigitta, 4333 Nyirkáta (HU)
(74) Representative: Best, Michael

(57) **Abstract**

The present invention relates to a pharmaceutical composition for prolonged release of nevirapine, comprising nevirapine or a pharmaceutically acceptable salt thereof as the sole pharmaceutically active ingredient and polyethylene oxide as a matrix-forming component, wherein the amount of the polyethylene oxide with at least 20 wt.-%, based on the total weight of the pharmaceutical composition.

## Description

### FIELD OF THE INVENTION

The present invention lies in the technical field of pharmaceutical compositions and formulations for prolonged release of nevirapine, and processes for manufacturing same

### BACKGROUND OF THE INVENTION

Nevirapine, or 11-cyclopropyl-5,11-dihydro-4-methyl-6H-dipyrido[3,2-b:2',3'-e][1,4]-diazepin-6-one, is a well-known agent for the treatment of infection caused by HIV-1 (human immunodeficiency virus, type 1), which acts through specific inhibition of HIV-1 reverse transcriptase. Its synthesis and use are described in various publications including, inter alia, US 5,366,972 and EP 0 429 987 B1.

Nevirapine is currently marketed as Viramune® tablets, wherein nevirapine is either formulated in an immediate release formulation for administration twice daily or as an extended release formulation, wherein nevirapine is formulated in an extended release matrix formed from hydroxypropylmethylcellulose (HPMC), also known as hypromellose, as hydrophilic polymer which imparts extended release of the nevirapine.

An extended release formulation with the active ingredient nevirapine is disclosed in the international patent application document WO 2008/154234 A9, wherein a nevirapine formulation comprising between about 20 and 25% by weight of hypromellose is disclosed as the preferred formulation, allowing administration only once per day while still maintaining a therapeutically appropriate blood level of the active ingredient. In all examples of WO 2008/154234 A9 nevirapine is formulated in an extended release matrix formed from hydroxypropylmethylcellulose.

A pharmaceutical composition comprising a combination of the antiretrovirally active agents lamivudine, festinavir, and nevirapine is disclosed in WO 2013/132208 A1. Said antiretroviral composition is provided in an oral dosage unit such as a multilayer tablet for once or twice daily administration wherein the nevirapine component may be formulated in a separate layer together with at least one hydrophilic and/or hydrophobic and/or water swellable polymer in order to allow extended release of nevirapine.

The prior art documents, in particular WO 2008/154234 A9, disclose a high number of possible matrix materials for the prolonged release of nevirapine. However, in practice only hydroxypropylmethylcellulose has been used and, if other materials are used, it is seen that the dissolution of nevirapine from the corresponding formulations is unsatisfactory and significantly different from the advantageous dissolution profile of the hydroxypropylmethylcellulose formulations. With these other polymeric matrix materials it is not possible to obtain formulations which are bioequivalent to the commercial Viramune^{®} retard formulation.

In view of the above, there thus remains a need for the provision of further prolonged release compositions and formulations, respectively, of nevirapine having an advantageous dissolution profile of nevirapine, and in particular having a similar dissolution profile compared to the commercial product Viramune^{®} retard. Preferably, the new prolonged release formulation of nevirapine should have a bioavailability which corresponds to the commercial product Viramune^{®} retard.

Said object is solved herein by a pharmaceutical composition for prolonged release of nevirapine, comprising nevirapine or a pharmaceutically acceptable salt thereof as the sole pharmaceutically active ingredient and polyethylene oxide as a matrix-forming component, wherein the amount of the polyethylene oxide is at least 20 wt.-%, based on the total weight of the pharmaceutical composition, and a pharmaceutical formulation comprising said pharmaceutical composition.

### BRIEF DESCRIPTION OF THE FIGURES

**Figure 1** shows the dissolution profiles of nevirapine in pharmaceutical prolonged release formulations of nevirapine comprising different polymer matrices.
**Figure 2** shows the dissolution profiles of nevirapine in pharmaceutical prolonged release formulations of nevirapine comprising different amounts of polyethylene oxide as the polymer matrix.
**Figure 3** shows the dissolution profiles of nevirapine in pharmaceutical prolonged release formulations of nevirapine comprising two polyethylene oxides with different molecular weights in different amounts.

### DETAILED DESCRIPTION OF THE INVENTION

### Definitions

The term "pharmaceutical composition", as used herein, refers to a composition comprising pharmaceutically active ingredients and pharmaceutically acceptable excipients providing a therapeutic effect for the treatment and prevention of a disease in a patient.

The term "pharmaceutically active ingredient", as used herein, refers to an ingredient of the claimed pharmaceutical composition which is included in the pharmaceutical composition in order to provide a therapeutic effect, in particular in order to treat or prevent a disease of a patient. Thus, a pharmaceutically active ingredient in the pharmaceutical composition is not a pharmaceutically acceptable excipient.

The term "sole pharmaceutically active ingredient", as used herein, implies that the pharmaceutical composition and formulation of the present invention solely contains nevirapine or a pharmaceutically active salt thereof as the pharmaceutically active ingredient. Further pharmaceutically active ingredients, i.e. ingredients which provide a therapeutic effect, in particular in order to treat or prevent a disease in a patient, are thus excluded from the pharmaceutical composition of the present invention.

The term "pharmaceutical formulation", as used herein, refers to a dosage form of the pharmaceutical composition of the invention being suitable for administering said pharmaceutical composition to a patient.

The term "release", as used herein, refers to the release of the pharmaceutically active ingredient from a pharmaceutical formulation comprising the pharmaceutical composition of the invention after exposing same to conditions whereupon the pharmaceutically active ingredient is released from the pharmaceutical formulation.

The term "prolonged release", as used herein, refers to the release of the pharmaceutically active ingredient from a pharmaceutical formulation comprising the pharmaceutical composition over a prolonged (extended) period of time compared to an immediate release formulation of said pharmaceutically active ingredient. "Prolonged release", as used herein, may be considered equivalent to the term "extended release".

The term "dissolution profile", as used herein, refers to the in-vitro dissolution rate of the pharmaceutically active ingredient from a pharmaceutical formulation comprising the pharmaceutical composition of the invention, the dissolution rate being measured by the Ph.Eur. Apparatus II (Paddle) equipment at 75 rpm in 900 ml 0.1 M HCl at 37°C. The dissolution is measured by measuring the amount of nevirapine dissolved (released) form the tested formulation at each time period.

The term "similar dissolution profile", as used herein in particular when comparing a dissolution profile of a pharmaceutical formulation comprising the pharmaceutical composition of the present invention to the commercial product Viramune^{®} retard, implies that the nevirapine is released from the formulation in a similar rate (percentage amount/time period). In this regard, "similar" means a deviation from the reference dissolution profile of Viramune^{®} retard of ±15%, preferably ± 10%, at each time point (e.g., if at 10 hours 50% of nevirapine are released, the 15% cover the range from 35% to 65% of nevirapine released).

The abbreviation "wt.-%", as used herein, means "weight percentage", and refers to the weight amount of a component (ingredient) of the pharmaceutical composition or formulation in relation to the total (overall) weight amount of the pharmaceutical composition or formulation if nothing else is explicitly stated or obvious under the circumstances.

The term "molecular weight" or "Mw", as used herein, refers to the weight average molecular weight of the corresponding polymer, as determined by GPC if nothing else is explicitly stated or obvious under the circumstances.

The use of the term "about", in particular in conjunction with the molecular weight (Mw) of the polymer, attempts to address the fact that the molecular weight of a polymer cannot exactly be determined and underlies measurement fluctuations. For the purpose of the present invention, the term "about" used in conjunction with the molecular weight (Mw) of a polymer thus refers the molecular weight (Mw) of the polymer ± 20%, preferably ± 10%.

### Pharmaceutical Composition according to the Invention

The pharmaceutical composition for prolonged release of nevirapine according to the present invention allows the adjustment of the dissolution profile of nevirapine in a pharmaceutical formulation comprising the pharmaceutical composition of the present invention. This is particularly achieved by using polyethylene oxide as a polymer component in the pharmaceutical composition according to the present invention in an amount of at least 20 wt.-%, based on the total weight of the pharmaceutical composition according to the present invention. In a particularly preferred embodiment, a pharmaceutical formulation containing the pharmaceutical composition of the present invention has a dissolution profile of nevirapine which is similar to the dissolution profile of nevirapine in the commercial product Viramune^{®} retard.

For the purpose of the present invention, the sole pharmaceutically active ingredient in the pharmaceutical composition and formulation according to the present invention is nevirapine or a pharmaceutically acceptable salt thereof. Accordingly, the therapeutically effect of the pharmaceutical composition of the present invention and the formulation comprising the pharmaceutical composition of the present invention is only related to nevirapine or a pharmaceutically acceptable salt thereof. Further pharmaceutically active ingredients are excluded from the pharmaceutical composition and formulation of the present invention. In particular, the pharmaceutical compositions and formulations of the present invention do not contain festinavir and/or lamivudine.

The pharmaceutically active ingredient which is present in the pharmaceutical composition of the present invention is nevirapine or a pharmaceutically acceptable salt thereof. This term includes the anhydrate form and all hydrated forms of nevirapine and of the pharmaceutically acceptable salts thereof. The pharmaceutically active ingredient is preferred nevirapine as free base either as anhydrate or as hemihydrate or in the form of its hydrochloride salt. Also, mixtures of nevirapine or a pharmaceutically acceptable salt thereof are not excluded, and may be used in the pharmaceutical composition of the present invention. Nevirapine in form of its free base and here in particular in anhydrous form is particularly preferred.

The amount of the pharmaceutically active ingredient in the pharmaceutical composition of the present invention is preferably 5 to 60 wt.-%, more preferably 10 to 55 wt.-%, even more preferably 15 to 50 wt.-%, and particularly preferably 20 to 45 wt.-%, each based on the total weight of the pharmaceutical composition.

In the pharmaceutical composition of the present invention, prolonged release of nevirapine or a pharmaceutically acceptable salt thereof is achieved by the polyethylene oxide used as the polymer component in the pharmaceutical composition. It is not excluded in the present invention that further polymers are contained in the pharmaceutical composition of the present invention which also have the ability to contribute to a prolonged release of the pharmaceutically active ingredient. However, further polymers besides the polyethylene oxide are not essential in the pharmaceutical composition of the present invention and are preferably not present.

Polyethylene oxides, as used in the pharmaceutical composition of the present invention, are nonionic, hydrophilic polymers, which hydrate and swell rapidly upon exposure to water or gastric juices to form hydrogels with properties ideally suited for prolonged-release of nevirapine or a pharmaceutically acceptable salt thereof. They also have the advantage that they are available in various molecular weight (Mw) grades so that the dissolution profile of nevirapine or a pharmaceutically acceptable salt thereof in a pharmaceutical formulation comprising the pharmaceutical composition of the present invention can be adjusted by selecting a polyethylene oxide of a particular molecular weight (Mw) in a particular amount. Polyethylene oxides furthermore do not interact with the pharmaceutically active ingredient nevirapine so that the dissolution profile of nevirapine can be adjusted in a reproducible manner. Finally, polyethylene oxides are cheap and readily available. Examples for commercial polyethylene oxides include POLYOX Water-soluble Resins marketed by The Dow Chemical Company.

In a preferred embodiment, the pharmaceutical composition of the present invention is provided as a matrix-based prolonged release composition, wherein the polyethylene oxide forms the matrix of the matrix-based formulation of the pharmaceutical composition of the present invention, thereby allowing a prolonged release of nevirapine from the pharmaceutical formulation.

The amount of the polyethylene oxide in the pharmaceutical composition according to the present invention influences the dissolution profile of the nevirapine in a pharmaceutical formulation comprising the pharmaceutical composition of the present invention. As a general trend, a higher percentage amount of the polyethylene oxide in the pharmaceutical composition according to the present invention slows down the release of nevirapine from a formulation comprising said composition compared to a qualitatively similar formulation comprising a lower percentage amount of said polyethylene oxide, whereas a lower percentage amount of the polyethylene oxide in a pharmaceutical formulation comprising the pharmaceutical composition according to the present invention accelerates release of nevirapine compared to a qualitatively similar formulation comprising a lower percentage amount of said polyethylene oxide. In general, the amount of the polyethylene oxide in the pharmaceutical composition according to the present invention should not exceed 60 wt.-%, preferably 58 wt.-%, more preferably 56, even more preferably 54, and particularly preferably 52 wt.-% based on the total weight of the pharmaceutical composition.

As noted above, also the molecular weight (Mw) of the polyethylene oxide influences the dissolution profile of nevirapine in a formulation comprising the pharmaceutical composition of the present invention. Thus, for obtaining an advantageous dissolution profile of nevirapine, a polyethylene oxide with a suitable weight average molecular weight should be used.

In a preferred embodiment of the present invention, the polyethylene oxide has a molecular weight (Mw) of 700,000 to 9,000,000. More preferably, the molecular weight (Mw) of the polyethylene oxide ranges from 800,000 to 8,000,000, and particularly preferable is a molecular weight (Mw) of the polyethylene oxide of 900,000 to 7,000,000.

In yet another preferred embodiment of the present invention, the polyethylene oxide has a high molecular weight (Mw) of 5,000,000 to 9,000,000. By using a high molecular weight polyethylene oxide having a molecular weight of Mw 5,000,000 to 9,000,000 in an amount of 20 to 30 wt.-%, based on the total weight of the pharmaceutical composition according to the present invention, an advantageous dissolution profile of nevirapine can be obtained, which is similar to the nevirapine dissolution profile in the commercial product Viramune^{®} retard.

In a further preferred embodiment of the present invention, the polyethylene oxide used in the composition of the present invention is a mixture of at least two polyethylene oxides with different molecular weights (Mw). Preferably, one of the at least two polyethylene oxides has a low molecular weight (Mw) of 700,000 to 1,100,000 ("low molecular weight polyethylene oxide"), and the other one of the at least two polyethylene oxides has a high molecular weight (Mw) of 5,000,000 to 9,000,000 ("high molecular weight polyethylene oxide"). In an even more preferred embodiment of the present invention, the polyethylene oxide is a mixture of at least two polyethylene oxides with different molecular weights, wherein the low molecular weight polyethylene oxide has a molecular weight (Mw) of 800,000 to 1,000,000 and the high molecular weight polyethylene oxide has a molecular weight (Mw) of 6,000,000 to 8,000,000. In a particularly preferred embodiment of the present invention, the pharmaceutical composition comprises a low molecular weight polyethylene oxide having a molecular weight (Mw) of about 900,000 and a high molecular weight polyethylene oxide has a molecular weight (Mw) of about 7,000,000.

The mixture of at least two polyethylene oxides with different molecular weights in the pharmaceutical composition of the present invention allows the adjustment of the desired dissolution profile of nevirapine. Moreover, the respective amounts of the high molecular weight polyethylene oxide and the low molecular weight polyethylene oxide influence the dissolution profile of nevirapine in a pharmaceutical formulation comprising the pharmaceutical composition of the present invention.

On account of the above, the low molecular weight polyethylene oxide is preferably used in the pharmaceutical composition according to the present invention in an amount of not more than 50 wt.-%, based on the total weight of the pharmaceutical composition, more preferably not more than 47.5 wt.-%, even more preferably not more than 45 wt.-%, even more preferably not more than 42.5 wt.-%, even more preferably not more than 40 wt.-%, and particularly preferably not more than 35 wt.-%, based on the total weight of the pharmaceutical composition.

The high molecular weight polyethylene oxide is preferably used in the pharmaceutical composition according to the present invention in an amount of at least 2.5 wt.-%, based on the total weight of the pharmaceutical composition, more preferably at least 5 wt.-%, even more preferably at least 7.5 wt.-%, even more preferably at least 10 wt.-%, and particularly preferably at least 15 wt.-%, based on the total weight of the pharmaceutical composition.

If the polyethylene oxide in the pharmaceutical composition only is high molecular weight polyethylene oxide, the preferred amount of polyethylene oxide in the composition is 20 to 30%, more preferably 20%.

It is noted that the overall amount of polyethylene oxide in the pharmaceutical composition according to the present invention should not exceed 60 wt.-%, preferably 58 wt.-%, more preferably 56 wt.-%, even more preferably 54 wt.-%, and particularly preferably 52 wt.-%, based on the total weight of the pharmaceutical composition as indicated above.

In a particularly preferred embodiment of the pharmaceutical composition according to the present invention, the pharmaceutical composition comprises 35 wt.-% of a polyethylene oxide with a molecular weight (Mw) of about 900,000, and 15 wt.-% of a polyethylene oxide with a molecular weight (Mw) of about 7,000,000, each based on the total weight of the pharmaceutical composition. These amounts and molecular weights (Mw) of the low molecular weight polyethylene oxide and the high molecular weight polyethylene oxide in the pharmaceutical composition according to the present invention are particularly advantageous in order to obtain a dissolution profile for nevirapine in a pharmaceutical formulation comprising the pharmaceutical composition of the present invention, which is similar to the dissolution profile of nevirapine in the commercial product Viramune^{®} retard and which provides a comparable bioavailability.

The pharmaceutical composition according to the present invention can include one or more pharmaceutically acceptable excipients, which are selected e.g. from the group consisting of carriers, fillers, diluents, glidants, lubricants, surfactants, and binders in addition to the active ingredient and the polyethylene oxide.

Pharmaceutically preferred carriers, fillers and diluents for use in the pharmaceutical composition according to the present invention include, but are not limited to, lactose, white sugar, lactitol, sucrose, saccharose, compressible sugars, sugar confectioners, glucose, calcium carbonate, calcium dihydrogen phosphate dihydrates, calcium phosphate-dibasic, calcium phosphate-tribasic, calcium sulfate, silicified macrocrystalline cellulose, cellulose powdered, fructose, kaolin sorbitol, mannitol, dextrates, dextrins, dextrose, maltodextrin, croscarmellose sodium, microcrystalline cellulose, hydroxypropylcellulose, L-hydroxypropylcellulose (low substituted), hydroxypropyl methylcellulose (HPMC), methylcellulose polymers, hydroxyethylcellulose, sodium carboxymethylcellulose, carboxymethylene, carboxymethyl hydroxyethylcellulose and other cellulose derivatives, starches or modified starches (including potato starch, corn starch, maize starch and rice starch) and any mixtures or combinations thereof.

Pharmaceutically acceptable surfactants for use in the pharmaceutical composition according to the present invention include, but are not limited to, polysorbates, sodium dodecyl sulfate (sodium lauryl sulfate), lauryl dimethyl amine oxide, docusate sodium, cetyltrimethyl ammonium bromide (CTAB), polyethoxylated alcohols, polyoxyethylenesorbitan, octoxynol, N,N-dimethyldodecylamine-N-oxide, hexadecyltrimethylammonium bromide, bile salts (sodium deoxycholate, sodium cholate), polyoxyl castor oil, nonylphenolethoxylate, cyclodextrins, lecithin, methylbenzethonium chloride, carboxylates, sulphonates, petroleum sulphonates, alkylbenzenesulphonates, naphthalenesulphonates, olefin sulphonates, alkyl sulphates, sulphates, sulphated natural oils and fats, sulphated esters, sulphatedalkanolamides, alkylphenols (ethoxylated and sulphated), ethoxylated aliphatic alcohol, polyoxyethylene surfactants, carboxylic esters polyethylene glycol esters, anhydrosorbitol ester (and ethoxylated derivatives thereof), glycol esters of fatty acids, carboxylic amides, monoalkanolamine condensates, polyoxyethylene fatty acid amides, quaternary ammonium salts, amines with amide linkages, polyoxyethylene alkyl and alicyclic amines, N,N,N,N-tetrakis-substituted ethylenediamines, 2-alkyl-1-hydroxyethyl 2-imidazolines, N-coco-3-aminopropionic acid/ sodium salt, N-tallow-3-iminodipropionate disodium salt, and any mixtures or combinations thereof.

Pharmaceutically acceptable glidants and lubricants for use in the pharmaceutical composition according to the present invention include but are not limited to, stearic acid and pharmaceutically acceptable salts or esters thereof (for example, magnesium stearate, calcium stearate, sodium stearyl fumarate or other metallic stearate), talc, waxes (for example, microcrystalline waxes), glycerides, light mineral oil, PEG, silica acid or a derivative or salt thereof (for example, silicates, silicon dioxide, colloidal silicon dioxide and polymers thereof, crospovidone, magnesium aluminosilicate and/ or magnesium alumino metasilicate), sucrose ester of fatty acids, hydrogenated vegetable oils (for example, hydrogenated castor oil) mineral oil, stearic acid, colloidal anhydrous silica, sucrose esters of fatty acids, microcrystalline wax, yellow beeswax, white beeswax and any mixtures or combinations thereof.

Pharmaceutically acceptable binders for use in the pharmaceutical composition according to the present invention include, but are not limited to, polyvinyl pyrrolidone (also known as povidone), polyethylene glycol(s), acacia, alginic acid, agar, calcium carragenan, cellulose derivatives such as ethyl cellulose, methyl cellulose, hydroxypropyl cellulose, hydroxypropyl methyl cellulose, sodium carboxymethylcellulose, dextrin, gelatin, gum arabic, guar gum, tragacanth, sodium alginate starches, corn starch, pregelatinized starch, microcrystalline celluloses (MCC), silicified MCC, microfine celluloses, lactose, calcium carbonate, calcium sulfate, sugar, mannitol, sorbitol, dextrates, dextrin, maltodextrin, dextrose, dibasic calcium phosphate dihydrate, tribasic calcium phosphate, magnesium carbonate, magnesium oxide, stearic acid, gums, hydroxypropyl methylcelluloses or hypromelloses and any mixtures or combinations thereof.

The one or more pharmaceutically acceptable excipients may preferably be included in the pharmaceutical composition according to the present invention in an amount of 1 to 60 wt.-%, based on the total weight of the pharmaceutical composition, more preferably 2 to 55 wt.-%, even more preferably 3 to 50 wt.-%, and particularly preferably 4 to 45 wt.-%, each based on the total weight of the pharmaceutical composition.

In a preferred embodiment of the present invention, the pharmaceutical composition is used in the treatment of HIV infections, particularly HIV-1 related HIV infections, and in particular AIDS. The pharmaceutical composition according to the present invention may further be used for the manufacture of a pharmaceutical formulation for use in the treatment of HIV infections, particularly HIV-1 related HIV infections, and in particular AIDS.

### Pharmaceutical Formulation comprising the Pharmaceutical Composition according to the Invention

The present invention further relates to a pharmaceutical formulation comprising the pharmaceutical composition according to the present invention.

The pharmaceutical composition according to the present invention may be formulated for oral, buccal, transdermal and subcutaneous administration. A correspondingly formulated composition is designated herein as pharmaceutical formulation. Thus, in one embodiment, the components of the pharmaceutical composition of the present invention and of the pharmaceutical formulation of the present invention are identical, but the pharmaceutical formulation is in a certain form that is suitable for administration to a patient.

Thus, a pharmaceutical formulation as this term is used herein is a pharmaceutical composition which is in a form that is suitable for direct administration to a patient. For example, a tablet which is suitable for administration to a patient is both a pharmaceutical formulation and pharmaceutical composition. A tablet mass that is to be compressed into tablets is a pharmaceutical composition but not yet a pharmaceutical formulation. A granulate as such is a pharmaceutical composition and can be a pharmaceutical formulation, if the granulate as such is suitable to be administered to a patient without first being compressed to tablets or filled e.g. into capsules.

In a preferred embodiment, which may be combined with any of the preceding or following embodiments of the present invention, the pharmaceutical formulation comprising the pharmaceutical composition of the present invention is provided as an oral dosage unit for oral administration of the pharmaceutical composition.

Said oral dosage unit may be in the form of a tablet, pill, capsule (filled with powder, pellets, beads, mini-tablets, pills, micro-pellets, small tablet units, granules, microspheres, multiparticulates), soft gelatin capsules, sachets (filled with powder, pellets, beads, mini-tablets, pills, micro-pellets, small tablet units, granules, microspheres, multiparticulates), sprinkles. In a particularly preferred embodiment of the present invention, the oral dosage unit is in the form of a tablet, pill or capsule, most preferably a tablet.

The pharmaceutical formulation comprising the pharmaceutical composition according to the present invention preferably comprises nevirapine in an amount of 50 to 600 mg, more preferably 100 to 550, even more preferably 200 to 500, even more preferably 300 to 450 mg. Particularly preferred is a formulation comprising the pharmaceutical composition according to the present invention which contains nevirapine in an amount of 400 mg. Said dosage of nevirapine allows an efficient pharmaceutical profile of nevirapine in the treatment of HIV infections, particularly HIV-1 related HIV infections, and in particular AIDS.

Preferably, the pharmaceutical formulation comprising the pharmaceutical composition according to the present invention represents a matrix-based formulation of nevirapine, wherein the polyethylene oxide forms the matrix of the matrix-based formulation, thereby allowing a prolonged release of nevirapine.

The pharmaceutical formulation of the present invention is preferably for administration to a patient once per day.

The pharmaceutical formulation comprising the pharmaceutical composition according to the present invention exhibits preferably an in-vitro dissolution profile of nevirapine wherein at least 2 wt.-% and not more than 20 wt.-% of the nevirapine or a pharmaceutically acceptable salt thereof, based on the total amount of nevirapine in the pharmaceutical formulation, is released at 2 hours; at least 20 wt.-% and not more than 80 wt.-% of the nevirapine or a pharmaceutically acceptable salt thereof, based on the total amount of nevirapine in the pharmaceutical formulation, is released at 8 hours; at least 40 wt.-% and not more than 98 wt.-% of the nevirapine or a pharmaceutically acceptable salt thereof, based on the total amount of nevirapine in the pharmaceutical formulation, is released at 12 hours; and at least 75 wt.-% up to 100 wt.-% of the nevirapine or a pharmaceutically acceptable salt thereof, based on the total amount of nevirapine in the pharmaceutical formulation, is released at 24 hours.

In a particularly preferred embodiment, the pharmaceutical formulation comprising the composition according to the present invention exhibits an in-vitro dissolution profile of nevirapine wherein at least 2 wt.-% and not more than 20 wt.-% of the nevirapine or a pharmaceutically acceptable salt thereof is released at 2 hours; at least 30 wt.-% and not more than 60 wt.-% of the nevirapine or a pharmaceutically acceptable salt thereof is released at 8 hours; at least 50 wt.-% and not more than 80 wt.-% of the nevirapine or a pharmaceutically acceptable salt thereof is released at 12 hours; at least 60 weight percent and not more than 90 wt.-% of the nevirapine or a pharmaceutically acceptable salt thereof is released at 16 hours; and at least 80 wt.-% up to 100 wt.-% of the nevirapine or a pharmaceutically acceptable salt thereof, based on the total amount of nevirapine in the pharmaceutical formulation, is released at 24 hours.

The pharmaceutical formulation comprising the pharmaceutical composition according to the present invention can be used in the treatment of HIV infections, particularly HIV-1 related HIV infections, and in particular AIDS.

The pharmaceutical formulations of the present invention contain nevirapine or pharmaceutically acceptable salts thereof as the sole pharmaceutically active ingredient but in general they are administered in combination with other pharmaceutical formulations which contain other pharmaceutically active ingredients. It is to be understood that the use of the pharmaceutical formulations of the present invention for the treatment of any disease and in particular in the treatment of AIDS includes the concomitant administration of the pharmaceutical formulations of the present invention with other medicaments. The term "concomitant administration" is meant to relate to any administration of two or more medicaments which leads to some medical effect which is not reached by the administration of only one of the two or more medicaments.

### Process for the Manufacture of the Pharmaceutical Composition of the Present Invention

The present invention furthermore relates to a process for the manufacture of a pharmaceutical composition according to the present invention.

Said process comprises the step of mixing nevirapine or a pharmaceutically acceptable salt thereof, the polyethylene oxide and optionally further pharmaceutically acceptable excipients, using e.g. a high shear mixer, resulting in the formation of a homogenous mixture of ingredients.

Said mixture is subsequently granulated using a granulation liquid, resulting in the formation of a granulation. Granulation can be carried out by spraying the granulation liquid on the mixture of ingredients.

The granulation liquid for the granulation step can be selected from the group consisting of water, methanol, ethanol, acidified ethanol, acetone, diacetone, polyols, polyethers, oils, esters, alkyl ketones, methylene chloride, isopropyl alcohol, butyl alcohol, methyl acetate, ethyl acetate, isopropyl acetate, castor oil, ethylene glycol monoethyl ether, diethylene glycol monobutyl ether, diethylene glycol monoethyl ether, dimethylsulphoxide, N,N-dimethylformamide, tetrahydrofurane, and mixtures thereof. In a preferred embodiment of the present invention, the granulation liquid is water or ethanol.

Particularly preferred as the granulation liquid is ethanol since it contributes to the formation of a granulation, which has improved physical attributes, good flowability and compressibility, avoiding the behavior of sticking to the wall of the high shear mixer, making it easier to clean.

The granulation product is subsequently dried in a following step.

In order to obtain a pharmaceutical formulation comprising the pharmaceutical composition according to the present invention, the dried granulation product may optionally be milled and subsequently compressed into a tablet. The tablet may optionally be coated with a film-forming material.

Suitable film-forming agents include, but are not limited to, cellulose derivatives, such as, soluble alkyl- or hydroalkyl-cellulose derivatives such as methylcelluloses, hydroxymethyl celluloses, hydroxyethyl celluloses, hydroxypropyl celluloses, hydroxymethylethyl celluloses, hydroxypropyl methylcelluloses, sodium carboxymethyl celluloses, insoluble cellulose derivatives such as ethylcelluloses and the like, dextrins, starches and starch derivatives, polymers based on carbohydrates and derivatives thereof, natural gums such as gum Arabic, xanthans, alginates, polyacrylic acids, polyvinyl alcohols, polyvinyl acetates, polyvinylpyrrolidones, polymethacrylates and derivatives thereof, chitosan and derivatives thereof, shellac and derivatives thereof, waxes, fat substances and any mixtures or combinations thereof. The film should not significantly change the release of nevirapine from the pharmaceutical formulations of the present invention.

Alternatively, the dried granulation product may directly be filled into a capsule.

For example, the process can be carried out as follows:
a. Dissolve Povidone (K-25) in Ethanol 96 per cent Portion I.
b. Transfer Nevirapine anhydrous and Cellulose microcrystalline (Type 101) into the High Shear Mixer and blend them.
c. Granulate the dry powder mix with the solution from step a).
d. Continue the granulation with Ethanol 96 per cent Portion II to rinse the vessel of solution from step a).
e. Dry the material to the required LOD (<1.5%).
f. Pass the dry granulate through the Rotating impeller and transfer to the blender.
g. Weigh the granule and calculate the yield.
h. Pass Polyethylene Oxide (e.g. Sentry Polyox WSR 303 LEO) through a screen then transfer to the blender used in step f).
i. Blend the mixture of Polyethylene Oxide (e.g. Sentry Polyox WSR 1105 LEO) Portion I and Silica, colloidal anhydrous in a bag and pass through a screen then transfer to the blender used in step f).
j. Pass Polyethylene Oxide (e.g. Sentry Polyox WSR 1105 LEO) Portion II through a screen then transfer to the blender used in step f).
k. Mix the materials in the blender for 15 minutes.
l. Pass Magnesium stearate through a screen, blend the mixture of the sieved magnesium stearate and a part of the intermediate blend from step k), and transfer to the blender used in step k).
m. Mix the materials in the blender for 5 minutes.
n. Weigh the granule and calculate the yield.
o. Compress the granule into tablets.
p. Weigh the tablets and calculate the yield.
q. The tablets are packaged according to production requirements.

Typical process parameters for the process are listed below:
Step I - Dissolving
   a) mixing - high shear mixer
      mixing time until clear solution;
Step II - Initial blending
   b) mixing
      mixing time 300 sec;
Step III - Granulation
   c) granulation and d) rinsing
      granulation time 30 sec
   e) drying - fluid bed drier, atmospheric pressure inlet air temperature 50 °C
      endpoint: product temperature 42 °C
   f) milling - rotating impeller
      screen size 1.0 mm;
Step IV - Intermediate blending
   h, i, j) sieving - vibratory sieve screen size 0.8 mm
   k) mixing
      mixing time 15 min;
Step V - Final blending
   I) sieving
      screen size 0.4 mm
   m) mixing
      mixing time 5 min.

Granulation liquid (ethanol):
in case of 540 g batches: Portion I is 30 g, Portion II is 4.16 g, respectively;
in case of 5400 g batches: Portion I is 300 g, Portion II is 41 g respectively.

Specification of the machines:
for smaller batches:
   - granulation and drying: Diosna PVAC 10 high-shear mixer and Glatt GPCG 2 fluid bed dryer
   - milling: Quadro Comil rotating mill
   - pressing: Piccola D-C press machine;
for bigger batches:
   - granulation and drying: Diosna P 25 high-shear mixer and Diosna CAP 25 fluid bed dryer
   - milling: Quadro Comil rotating mill
   - pressing: Piccola D-C or Fette P1200 press machine.

A typical example of a tablet produced according to the present invention is e.g. as follows:

| **Product** | **Nevirapine 400 mg PR Tablets** |
|---|---|
| **Weight (mg)** | 1080 |
| **Shape** | Oval, biconvex |
| **Dimensions (mm)** | 20.5 x 10.0 |
| **Colour** | White to off-white (Pantone N/A) |
| **Score** | unscored |
| **Marked with** | 400 on one side, plain on the other side |
| **Packaging material (Size of packaging)** | HDPE container (60 ml) with a child resistant, tamper evident plastic cap and 2g of desiccant in the cap (30x) and OPA/Alu/Pvc-Alu blister |

### EXAMPLES

### Example 1: Comparison of the nevirapine dissolution profiles in pharmaceutical formulations with different polymers

Formulations **1a** to **1e** were obtained by mixing nevirapine anhydrous, the polymer component, and pharmaceutically acceptable excipients in the amounts indicated in the following Tables **1a** to **1e,** granulating the resulting mixture with water in a high shear mixer, drying the granulation product and compressing it into a tablet. The process conditions were as indicated above.

**Table 1a: Nevirapine/Carbomers formulation (Formulation 1a) (500 tablets/540 g)**

| **Ingredients** | **wt.-%** | **mg/tablet** |
|---|---|---|
| Nevirapine anhydrous | 37.04 | 400.0 |
| Mannitol | 29.96 | 323.6 |
| Iron oxide, yellow | 1.00 | 10.8 |
| **Carbomers (Carbopol 71G)** | **30.00** | **324.0** |
| Magnesium stearate | 2.00 | 21.6 |
| **Total** | 100.00 | 1080.0 |

**Table 1b: Nevirapine/Xanthan Gum formulation (Formulation 1b) (500 tablets/540 g)**

| **Ingredients** | **wt.-%** | **mg/ tablet** |
|---|---|---|
| Nevirapine anhydrous | 37.04 | 400.0 |
| Mannitol | 24.96 | 269.6 |
| Povidone K25 | 5.00 | 54.0 |
| Iron oxide, yellow | 1.00 | 10.8 |
| **Xanthan Gum** | **30.00** | **324.0** |
| Magnesium stearate | 2.00 | 21.6 |
| **Total** | 100.00 | 1080.0 |

**Table 1c: Nevirapine/Glycerol dibehenate formulation (Formulation 1c) (500 tablets/540 g)**

| **Ingredients** | **wt.-%** | **mg/ tablet** |
|---|---|---|
| Nevirapine anhydrous | 37.04 | 400.0 |
| Mannitol | 24.96 | 269.6 |
| Povidone K25 | 5.00 | 54.0 |
| Iron oxide, yellow | 1.00 | 10.8 |
| **Glycerol dibehenate** | 30.00 | **324.0** |
| Neusilin | 2.00 | 21.6 |
| **Total** | 100.00 | 1080.0 |

**Table 1d: Nevirapine/Hydroxypropylcellulose formulation (Formulation 1d) (500 tablets/540 g)**

| **Ingredients** | **wt.-%** | **mg/ tablet** |
|---|---|---|
| Nevirapine anhydrous | 37.04 | 400.0 |
| Mannitol | 31.46 | 339.8 |
| Iron oxide, yellow | 0.50 | 5.4 |
| **Hydroxypropylcellulose (Klucel EXF)** | **30.00** | **324.0** |
| Magnesium Stearate | 1.00 | 10.8 |
| **Total** | 100.00 | 1080.0 |

**Table 1e: Nevirapine/Polyethylene Oxide formulation (Formulation 1e) (500 tablets/540 g)**

| **Ingredients** | **wt.-%** | **mg/ tablet** |
|---|---|---|
| Nevirapine anhydrous | 37.04 | 400.0 |
| Mannitol | 24.96 | 269.6 |
| Povidone K25 | 5.00 | 54.0 |
| Iron oxide, yellow | 1.00 | 10.8 |
| **Polyethylene Oxide (Polyox WSR 303)** | **30.00** | **324.0** |
| Magnesium Stearate | 2.00 | 21.6 |
| **Total** | 100.00 | 1080.0 |

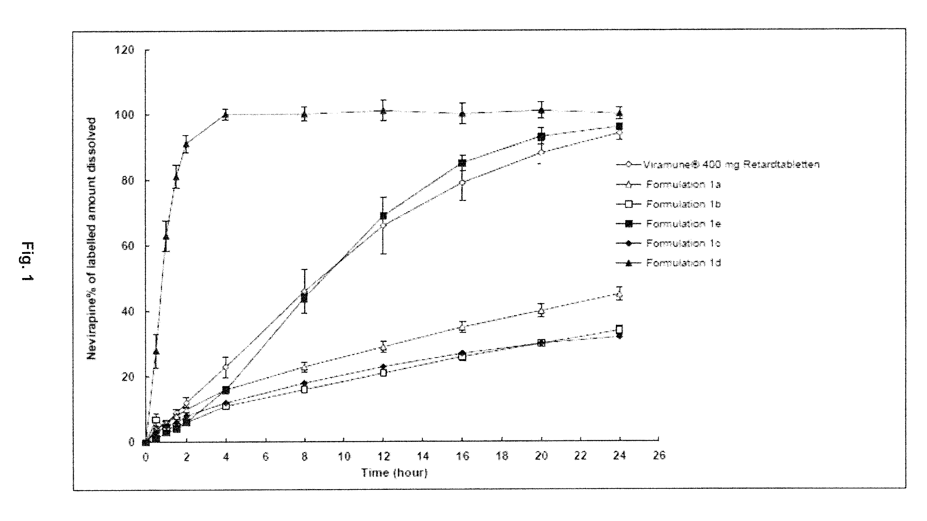

The dissolution profiles of the nevirapine formulations **1a** to **1e** have been determined in comparison to the dissolution profile of nevirapine in the commercial product Viramune^{®} retard, using the method as indicated above. The results are shown in **Figure 1****.**

As may be taken from **Figure 1****,** the polyethylene oxide formulation (Formulation **1e)** provides a dissolution profile of nevirapine, which, over a period of 24 hours, is similar to the dissolution profile of nevirapine in the commercial product Viramune^{®} retard, i.e. over a time period of 24 hours, the nevirapine is released in a similar rate (percentage amount/time period), and after time period of 24 hours upon beginning of the measurement, a similar percentage amount of nevirapine has been released.

The hydroxypropylcellulose formulation (Formulation **1d)** shows a significantly faster dissolution profile for nevirapine compared to the polyethylene oxide formulation (Formulation **1e)** and the commercial product Viramune^{®} retard. Contrary thereto, the carbomers formulation (Formulation **1a**), the xanthan gum formulation (Formulation **1b),** and the glycerol dibehenate formulation (Formulation **1c)** show a slower release profile for nevirapine compared to the polyethylene oxide formulation (Formulation **1e)** and the commercial product Viramune^{®} retard.

### Example 2: Comparison of the nevirapine dissolution profile in pharmaceutical formulations with different polyethylene oxide contents

Formulations **2a** to **2c** were obtained by mixing nevirapine anhydrous, the polyethylene oxide, and pharmaceutically acceptable excipients in the amounts indicated in the following Tables **2a** to **2c,** granulating the resulting mixture with ethanol in a high shear mixer, drying the granulation product, and compressing it into a tablet. The process conditions were as indicated above.

**Table 2a: Nevirapine/ 20 wt.-% Polyethylene Oxide formulation (Formulation 2a) (5000 tablets/5400 g)**

| **Ingredients** | **wt.-%** | **mg/tablet** |
|---|---|---|
| Nevirapine anhydrous | 37.04 | 400.0 |
| Povidone K25 | 4.00 | 43.2 |
| Cellulose microcrystalline | 38.21 | 412.7 |
| **Polyethylene Oxide (Polyox WSR 303)** | **20.00** | **216.0** |
| Silcia colloidal anhydrous | 0.25 | 2.7 |
| Magnesium Stearate | 0.50 | 5.4 |
| **Total** | 100.00 | 1080.0 |

**Table 2b: Nevirapine/ 25 wt.-% Polyethylene Oxide formulation (Formulation 2b) (5000 tablets/5400 g)**

| **Ingredients** | **wt.-%** | **mg/tablet** |
|---|---|---|
| Nevirapine anhydrous | 37.04 | 400.0 |
| Povidone K25 | 4.00 | 43.2 |
| Cellulose microcrystalline | 33.21 | 358.7 |
| **Polyethylene Oxide (Polyox WSR 303)** | **25.00** | **270.0** |
| Silcia colloidal anhydrous | 0.25 | 2.7 |
| Magnesium Stearate | 0.50 | 5.4 |
| **Total** | 100.00 | 1080.0 |

**Table 2c: Nevirapine/ 30 wt.-% Polyethylene Oxide formulation (Formulation 2c) (5000 tablets/5400 g)**

| **Ingredients** | **wt.-%** | **mg/tablet** |
|---|---|---|
| Nevirapine anhydrous | 37.04 | 400.0 |
| Povidone K25 | 4.00 | 43.2 |
| Cellulose microcrystalline | 28.21 | 304.7 |
| **Polyethylene Oxide (Polyox WSR 303)** | **30.00** | **324.0** |
| Silcia colloidal anhydrous | 0.25 | 2.7 |
| Magnesium Stearate | 0.50 | 5.4 |
| **Total** | 100.00 | 1080.0 |

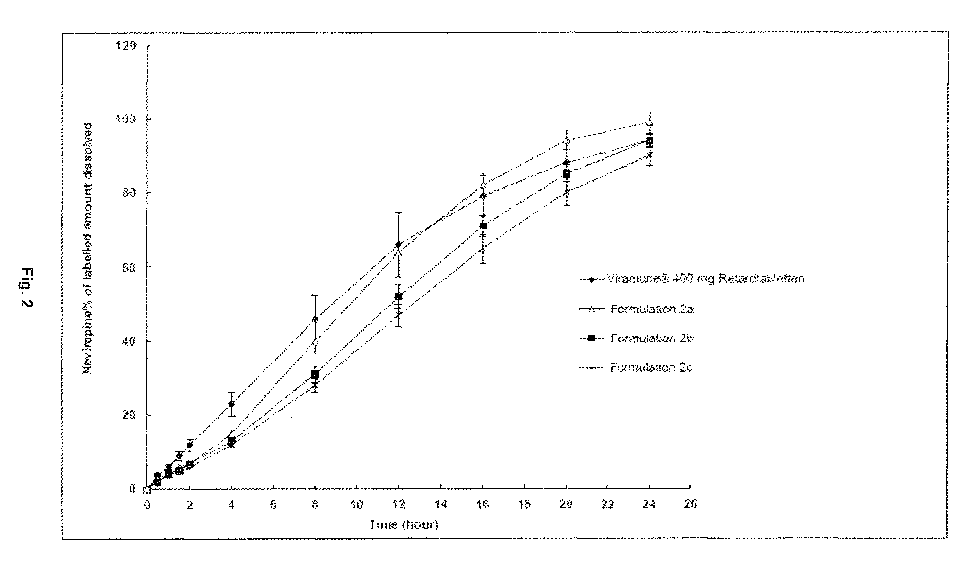

The dissolution profiles of the nevirapine formulations **2a** to **2c** have been determined in comparison to the release profile of nevirapine in the commercial product Viramune^{®} retard, using the method indicated above. The results are shown in **Figure 2****.**

As can be taken from **Figure 2****,** the dissolution profile of nevirapine is slowed down if the percentage amount of the polyethylene oxide component is increased in the formulation.

All three formulations **2a** to **2c** show that similar percentage amounts of nevirapine have been released after a time period of 24 hours after beginning of the measurement, compared to the commercial product Viramune^{®} retard. Formulations **2a** and **2b** (25 wt.-% Polyox WSR 303) show a similar dissolution profile compared to the commercial product Viramune^{®} retard over a time period of 24 hours. Formulation **2c** (30 wt.-% Polyox WSR 303) shows a somewhat slower dissolution profile of nevirapine over a time period of 24 hours compared to the commercial product Viramune^{®} retard, but the dissolution profile is still acceptable.

Formulation **2a** showed a particularly advantageous dissolution profile and passed a bioavailability test in a fed study.

### Example 3: Comparison of the nevirapine dissolution profile in pharmaceutical formulations with two polyethylene oxides with different molecular weights

Formulations **3a** to **3g** were obtained by mixing nevirapine anhydrous, the polyethylene oxide components, and the pharmaceutically acceptable excipients in the amounts indicated in the following Tables **3a** to **3g,** granulating the resulting mixture with ethanol in a high shear mixer, drying the granulation and compressing dried granulation into a tablet. The process conditions were as indicated above.

**Table 3a: Nevirapine/ 50 wt.-% Polyox WSR 1105 (Formulation 3a) (5000 tablets/5400 g)**

| **Ingredients** | **wt.-%** | **mg/tablet** |
|---|---|---|
| Nevirapine anhydrous | 37.04 | 400.0 |
| Povidone K25 | 4.00 | 43.2 |
| Cellulose microcrystalline | 8.21 | 88.7 |
| **Polyethylene Oxide (Polyox WSR 1105)** | **50.00** | **540.0** |
| **Polyethylene Oxide (Polyox WSR 303)** | **n**/**a** | **n**/**a** |
| Silcia colloidal anhydrous | 0.25 | 2.7 |
| Magnesium Stearate | 0.50 | 5.4 |
| **Total** | 100.00 | 1080.0 |

**Table 3b: Nevirapine/ 47.5 wt.-% Polyox WSR 1105/ 2.5 wt.-% Polyox WSR 303 (Formulation 3b) (5000 tablets/5400 g)**

| **Ingredients** | **wt.-%** | **mg/tablet** |
|---|---|---|
| Nevirapine anhydrous | 37.04 | 400.0 |
| Povidone K25 | 4.00 | 43.2 |
| Cellulose microcrystalline | 8.21 | 88.7 |
| **Polyethylene Oxide (Polyox WSR 1105)** | **47.50** | **513.0** |
| **Polyethylene Oxide (Polyox WSR 303)** | **2.50** | **27.0** |
| Silcia colloidal anhydrous | 0.25 | 2.7 |
| Magnesium Stearate | 0.50 | 5.4 |
| **Total** | 100.00 | 1080.0 |

**Table 3c: Nevirapine/ 45 wt.-% Polyox WSR 1105/ 5 wt.-% Polyox WSR 303 (Formulation 3c) (5000 tablets/5400 g)**

| **Ingredients** | **wt.-%** | **mg/tablet** |
|---|---|---|
| Nevirapine anhydrous | 37.04 | 400.0 |
| Povidone K25 | 4.00 | 43.2 |
| Cellulose microcrystalline | 8.21 | 88.7 |
| **Polyethylene Oxide (Polyox WSR 1105)** | **45.00** | **486.0** |
| **Polyethylene Oxide (Polyox WSR 303)** | **5.00** | **54.0** |
| Silcia colloidal anhydrous | 0.25 | 2.7 |
| Magnesium Stearate | 0.50 | 5.4 |
| **Total** | 100.00 | 1080.0 |

**Table 3d: Nevirapine/ 42.5 wt.-% Polyox WSR 1105/ 7.5 wt.-% Polyox WSR 303 (Formulation 3d) (5000 tablets/5400 g)**

| **Ingredients** | **wt.-%** | **mg/tablet** |
|---|---|---|
| Nevirapine anhydrous | 37.04 | 400.0 |
| Povidone K25 | 4.00 | 43.2 |
| Cellulose microcrystalline | 8.21 | 88.7 |
| **Polyethylene Oxide (Polyox WSR 1105)** | **42.50** | **459.0** |
| **Polyethylene Oxide (Polyox WSR 303)** | **7.50** | **81.0** |
| Silcia colloidal anhydrous | 0.25 | 2.7 |
| Magnesium Stearate | 0.50 | 5.4 |
| **Total** | 100.00 | 1080.0 |

**Table 3e: Nevirapine/ 40 wt.-% Polyox WSR 1105/ 10 wt.-% Polyox WSR 303 (Formulation 3e) (5000 tablets/5400 g)**

| **Ingredients** | **wt.-%** | **mg/tablet** |
|---|---|---|
| Nevirapine anhydrous | 37.04 | 400.0 |
| Povidone K25 | 4.00 | 43.2 |
| Cellulose microcrystalline | 8.21 | 88.7 |
| **Polyethylene Oxide (Polyox WSR 1105)** | **40.00** | **432.0** |
| **Polyethylene Oxide (Polyox WSR 303)** | **10.00** | **108.0** |
| Silcia colloidal anhydrous | 0.25 | 2.7 |
| Magnesium Stearate | 0.50 | 5.4 |
| **Total** | 100.00 | 1080.0 |

**Table 3f: Nevirapine/ 35 wt.-% Polyox WSR 1105/ 15 wt.-% Polyox WSR 303 (Formulation 3f) (5000 tablets/5400 g)**

| **Ingredients** | **wt.-%** | **mg/tablet** |
|---|---|---|
| Nevirapine anhydrous | 37.04 | 400.0 |
| Povidone K25 | 4.00 | 43.2 |
| Cellulose microcrystalline | 8.21 | 88.7 |
| **Polyethylene Oxide (Polyox WSR 1105)** | **35.00** | **378.0** |
| **Polyethylene Oxide (Polyox WSR 303)** | **15.00** | **162.0** |
| Silcia colloidal anhydrous | 0.25 | 2.7 |
| Magnesium Stearate | 0.50 | 5.4 |
| **Total** | 100.00 | 1080.0 |

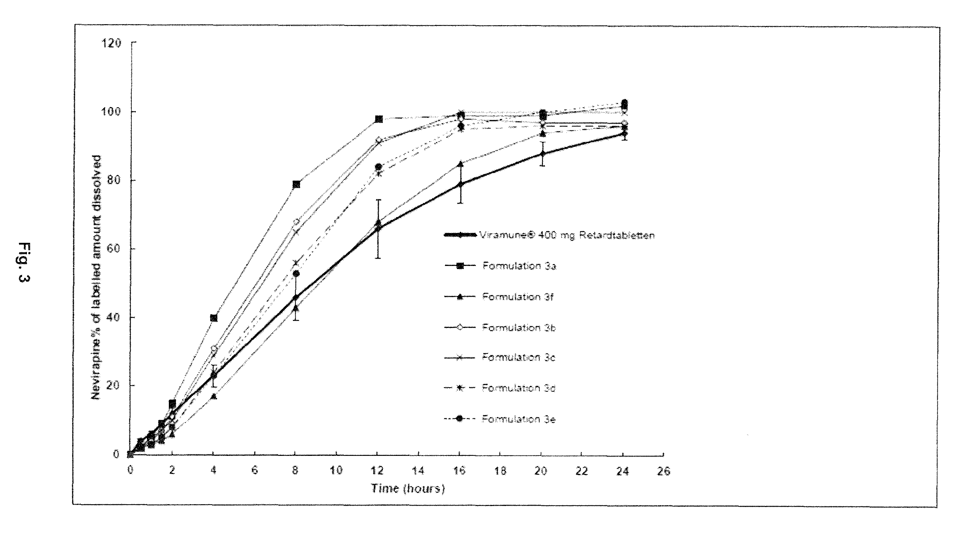

The dissolution profiles of the nevirapine formulations **3a** to **3f** have been determined in comparison to the release profile of nevirapine in the commercial product Viramune^{®} retard, using the method as indicated above. The results are shown in **Figure 3****.**

As can be taken from **Figure 3****,** the closest dissolution profile compared to the commercial product Viramune^{®} retard is achieved with formulation **3f,** which, over a time period of 24 hours upon beginning of the measurement, shows a similar release rate (percentage amount/time period) for nevirapine, and wherein after 24 hours upon beginning of the measurement, a similar percentage amount of nevirapine has been released from the formulation. Accordingly, formulation **3f** shows a similar dissolution profile for nevirapine in comparison with the dissolution profile of the commercial product Viramune^{®} retard.

## Claims

1. A pharmaceutical composition for prolonged release of nevirapine, comprising nevirapine or a pharmaceutically acceptable salt thereof as the sole pharmaceutically active ingredient and polyethylene oxide as a matrix-forming component, wherein the amount of the polyethylene oxide is at least 20 wt.-%, based on the total weight of the pharmaceutical composition.

2. The pharmaceutical composition according to claim 1, wherein the composition comprises a mixture of at least two polyethylene oxides with different molecular weights (Mw), wherein one of the at least two polyethylene oxides has a low molecular weight of Mw 700,000 to 1,100,000, and the other one of the at least two polyethylene oxides has a high molecular weight of Mw 5,000,000 to 9,000,000.

3. The pharmaceutical composition according to claim 2, wherein the polyethylene oxide with the low molecular weight is present in an amount of not more than 50 wt.-%, based on the total weight of the pharmaceutical composition.

4. The pharmaceutical composition according to claim 2 and/or 3, wherein the polyethylene oxide with the high molecular weight is present in an amount of at least 2.5 wt.-%, based on the total weight of the pharmaceutical composition.

5. The pharmaceutical composition according to any of the preceding claims, wherein the composition comprises 35 wt.-% of a polyethylene oxide with a molecular weight of Mw about 900,000, and 15 wt.-% of a polyethylene oxide with a molecular weight of Mw about 7,000,000, each based on the total weight of the pharmaceutical composition.

6. The pharmaceutical composition according to any of the preceding claims, wherein the composition further comprises one or more pharmaceutically acceptable excipients selected from the group consisting of carriers, fillers, diluents, glidants, lubricants, surfactants and binders.

7. The pharmaceutical composition according to any one of the preceding claims for use in the treatment of HIV infections, in particular of AIDS.

8. The pharmaceutical composition according to claim 7 for administration once every 24 hours.

9. A pharmaceutical formulation comprising the pharmaceutical composition according to claims 1 to 8, wherein said pharmaceutical formulation is provided as an oral dosage unit for oral administration and wherein nevirapine is the sole pharmaceutically active ingredient of the formulation.

10. A pharmaceutical formulation according to claim 9, wherein the oral dosage unit is in the form of a tablet, pill or capsule.

11. A pharmaceutical formulation according to claim 9 and/or 10, wherein said pharmaceutical formulation comprises 50 to 600 mg nevirapine or a pharmaceutically acceptable salt thereof.

12. A pharmaceutical formulation according to any of claims 9 to 11, wherein said pharmaceutical formulation exhibits an in-vitro dissolution profile wherein at least 2 wt.-% and not more than 20 wt.-% of nevirapine or a pharmaceutically acceptable salt thereof, based on the total amount of the nevirapine in the pharmaceutical formulation, is released at 2 hours; at least 20 wt.-% and not more than 80 wt.-% of the nevirapine or a pharmaceutically acceptable salt thereof, based on the total amount of nevirapine in the pharmaceutical formulation, is released at 8 hours; at least 40 wt.-% and not more than 98 wt.-% of the nevirapine or a pharmaceutically acceptable salt thereof, based on the total amount of nevirapine in the pharmaceutical formulation, is released at 12 hours; and at least 75 wt.-% up to 100 wt.-% of the nevirapine or a pharmaceutically acceptable salt thereof, based on the total amount of nevirapine in the pharmaceutical formulation, is released at 24 hours.

13. A process for the manufacture of a pharmaceutical composition according to any of claims 1 to 8, comprising the steps of mixing nevirapine or a pharmaceutically acceptable salt thereof, the polyethylene oxide and optionally further pharmaceutically acceptable excipients, resulting in a mixture; granulating said mixture with a granulation liquid, resulting in a granulation, and drying said granulation to obtain a dried granulation product.

14. The process according to claim 13, wherein the granulation liquid is ethanol.

15. A process for preparing a pharmaceutical formulation, comprising carrying out the process according to claims 13 and/or 14, further comprising compressing the dried granulation into a tablet or a pill and optionally coating the tablet or pill with a film-forming material, or filling the dried granulation product into a capsule.
